# EUROPEAN PATENT APPLICATION

(11) **EP 0 861 639 A2**
(43) Date of publication of application: **02.09.1998**
(21) Application number: 98300348.4
(22) Date of filing: 19.01.1998
(51) Int. Cl.: A61F 5/44, A61B 5/20

(54) **Urine bag assemblies**

(30) Priority: 13.02.1997 GB 9702987
(71) Applicant: Smiths Industries Public Limited Company, London, NW11 8DS (GB)
(72) Inventor: Batchelor, Kester Julian, Hythe, Kent CT21 6DA (GB)
(74) Representative: Flint, Jonathan McNeill

(57) **Abstract**

A urine bag assembly has a flexible bag 1 with a rigid meter 2 attached to its forward surface by means of a rotatable coupling 23, 56, 64 that permits rotation of the meter relative to the bag in the plane of the bag. The meter has an inlet 82 through which urine enters the assembly; the bag has an outlet 3 through which urine can be drained from the assembly. A passage through the coupling 23, 56, 64 between the meter 2 and the bag 1 enables the contents of the meter to be emptied periodically into the bag by swinging up the lower end of the meter.

## Description

This invention relates to urine bag assemblies of the kind including a flexible bag and a rigid meter with a urine inlet, the meter being attached with the bag and having an outlet connected to an inlet of the bag.

Following surgery, it is often necessary for a patient to be connected by a urinary catheter to a urine drainage bag, so that discharged urine can be collected for disposal. The bag may be graduated to give an approximate measure of the contents. Where a more accurate measure of discharged urine is needed, a urine bag assembly is used, which includes a urine meter in the form of a rigid, graduated container with an inlet connected to receive the discharged urine. The volume of the meter is less than that of the bag so it is necessary periodically to empty the contents of the meter into the bag. The meter may be mounted above the bag, such as described, for example, in WO 89/05119, WO 95/13016 or US 4579126. In such arrangements, the meter has an outlet with a tap at its lower end, which is opened to allow urine to drain from the meter to the bag. The length of such containers can be a disadvantage in some cases.

Alternatively, the meter may hang alongside the front of the bag, such as described, for example in GB2073595, GB 2191702, EP 471413, US 4699155, US 4301813 or US 4305405. In the latter arrangement, when the meter needs emptying, it is lifted above the bag and tipped so that its contents drain through an outlet into the bag. The meter may be clipped to the top of the bag and its outlet connected to the bag inlet by a flexible tube, the meter being unclipped from the bag before emptying. It can be awkward to have to unclip and reclip the meter each time after use. Alternatively, the top of the meter may be attached to the top of the bag and the meter emptied by pulling its lower end outwardly and upwardly away from the bag, so that its contents are tipped out through an outlet at the top of the meter. Where the meter is attached to the flexible material of the bag, the repeated lifting of the meter can result in damage to the bag material.

It is an object of the present invention to provide an improved urine bag assembly.

According to the present invention there is provided a urine bag assembly of the above-specified kind, characterised in that the meter is pivotally coupled with the bag so that it can be tipped about an axis extending normal to the plane of the bag to empty its contents into the bag

The outlet of the meter preferably connects with the inlet of the bag through the pivotal coupling. The bag preferably has a spigot projecting from its inlet, the meter having a hub surrounding the spigot and there being a seal between the spigot and the hub permitting rotation of the hub relative to the spigot. The bag may include a hanger, which may have a plate that extends on one side of the bag and a member folded over the other side of the bag, the plate and member having cooperating formations that engage with one another through a hole in the bag. The hanger preferably has an opening therethrough, the meter having a catch member that engages the opening and permits rotation of the meter relative to the hanger. The meter may have a catch on its rear surface that engages with a hook on the forward surface of the bag, engagement of the catch and hook limiting pivoting of the meter on the bag in one direction. The rear wall of the meter is preferably recessed at its lower end to accommodate filling of the bag. The lower end of the meter preferably has a smaller cross section than its upper end. The lower end of the bag may have a flexible outlet, the lower end of the bag having a recess to one side of the outlet so that the outlet can be bent up and retained in the recess.

A urine bag assembly according to the present invention, will now be described, by way of example, with reference to the accompanying drawings, in which:
- Figure 1: is an elevation view of the front of the assembly;
- Figure 2: is an elevation view of the front of bag, without the meter;
- Figure 3: is a cross-sectional view along a horizontal plane of a part of the assembly, to an enlarged scale, showing the fluid coupling between the meter and bag;
- Figure 4: is an elevation view of the front of a hanger forming a part of the assembly;
- Figure 5: is a plan view of the hanger in Figure 4;
- Figure 6: is a side elevation view of the hanger along the arrow VI of Figure 4;
- Figure 7: is a cross-sectional side elevation of a lower component of the meter;
- Figure 8: is a transverse sectional view of the component in Figure 7 along the line VIII-VIII of Figure 7;
- Figure 9: is a cross-sectional side elevation of an upper component of the meter; and
- Figures 10 and 11: are end elevation views illustrating the effect of filling of the bag.

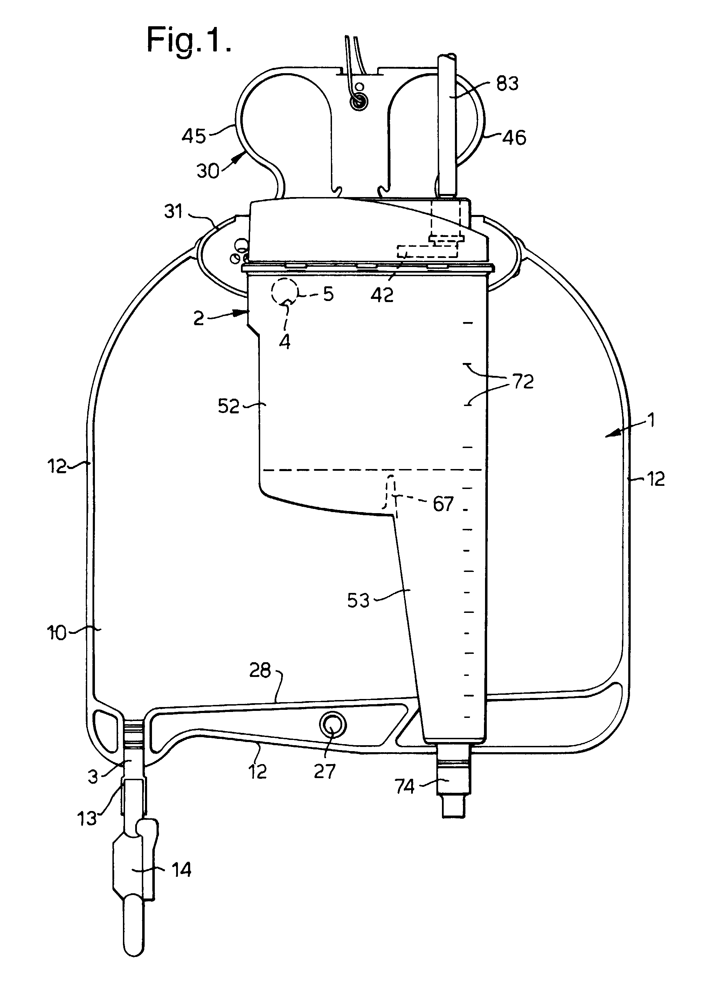

With reference first to Figure 1, the assembly includes a flexible bag 1 and a urine meter 2 hanging down in front of the bag. The bag 1 has an outlet 3 at its lower end and an inlet 4 towards its upper end, which is connected to the outlet 5 of the meter 2.

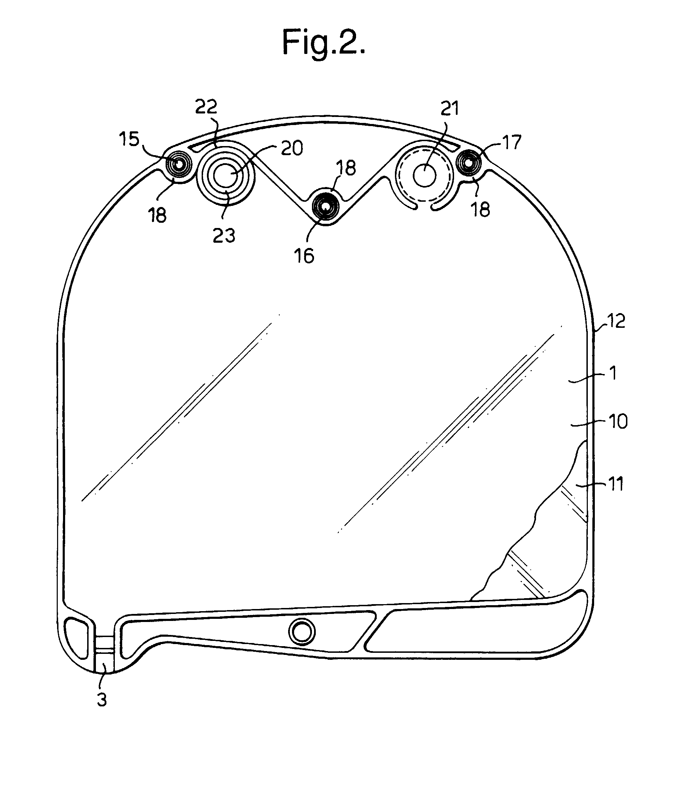

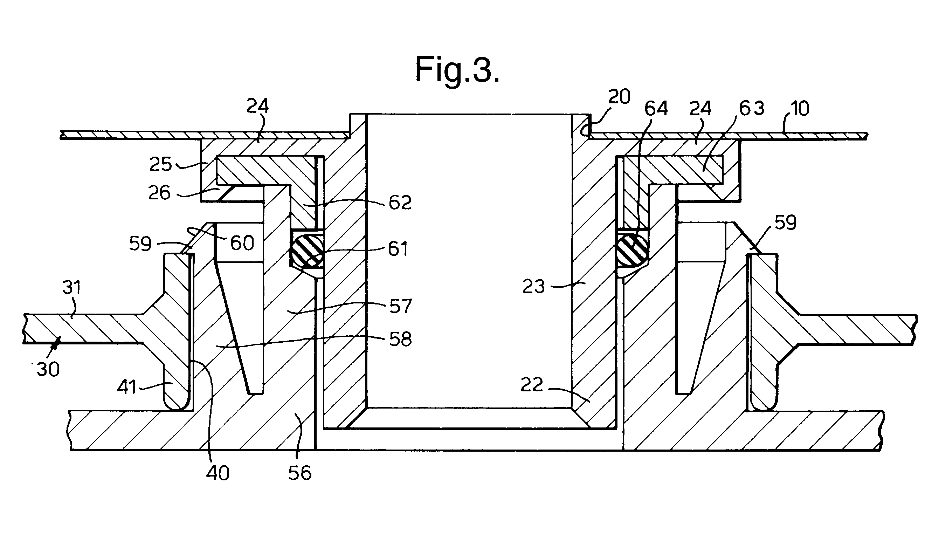

With reference now also to Figures 2 and 3, the bag 1 has a front and rear sheet 10 and 11 of PVC foil welded together around their outer edge 12. The outlet 3 takes the form of a short, flexible tube 13 welded between the two sheets at the lower left corner, the tube having a tap 14 by which the outlet can be opened and closed. The weld line along the lower edge of the bag slopes towards the left corner so that urine drains freely towards the outlet 3. At its upper end, the bag has three holes 15 to 17 punched through both sheets 10 and 11 and sealed from the interior of the bag by surrounding weld lines 18. Two of the holes 15 and 17 are located at opposite edges of the bag and the other hole 16 is located centrally; the holes serve to support the bag 1. The bag also has two further holes 20 and 21 located adjacent the outer support holes 15 and 17 respectively. These further holes 20 and 21 are formed through the front sheet 10 only and open into the interior of the bag. The left-hand hole 20 provides the inlet 4 to the bag 1 and its edge is welded to a circular connector 22 that projects outwardly of the bag. The connector 22 has a central spigot 23 and an outwardly-projecting flange 24 welded to the front face of the sheet 10. The flange 24 has a short rim 25 extending coaxially of the spigot 23 and having an internally-projecting tapered annular catch 26. The right-hand hole 21 serves as a vent outlet to allow gas to escape from the bag. The hole 21 is covered by a hydrophobic filter disc, which allows gas to escape but prevents the escape of liquid.

A hole 27 extends through the bottom of the bag 1, between the welded edge 12 and an inner weld line 28, so that the hole is isolated from the interior of the bag. The outlet tube 13 can be bent up and the free end of the tap 14 tucked into the hole 27. This reduces the overall length of the assembly and reduces the risk of the soiled end of the tap coming into contact with the surroundings.

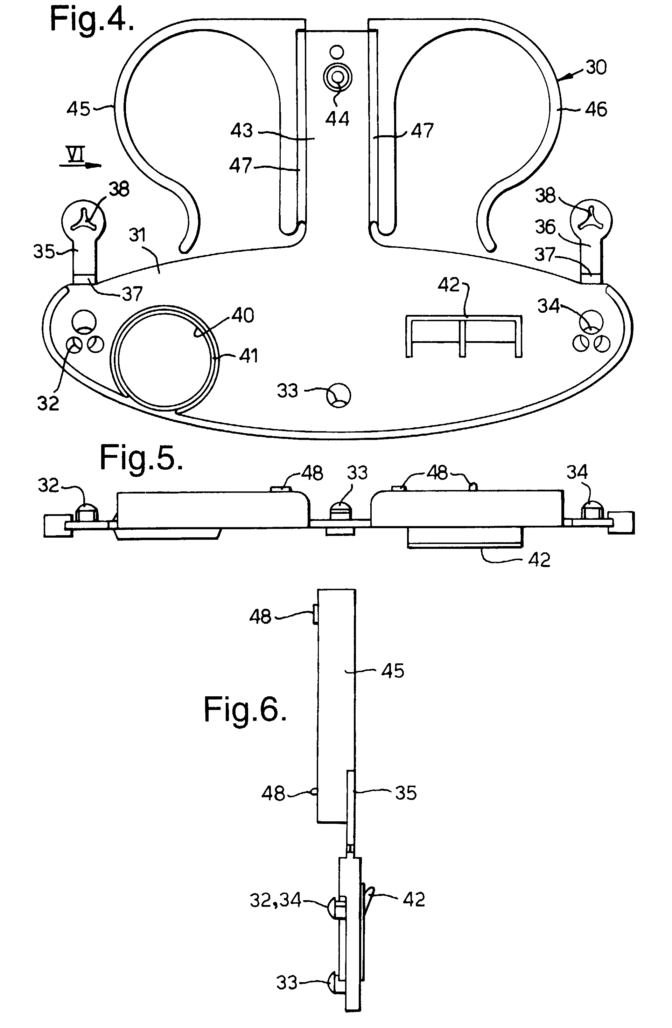

With reference now to Figures 4 to 6, the assembly also includes a hanger 30 moulded from a relatively stiff plastics, such as a polypropylene copolymer. The hanger 30 is a substantially flat component with a lower plate 31 of oval shape, which overlies the upper, front side of the bag 1. The plate 31 has three hooked studs 32 to 34 projecting from its rear face and located to align with the holes 15 to 17 respectively in the bag 1. The hanger 30 has two integral straps 35 and 36 extending vertically upwardly directly above the studs 32 and 34. The straps 35 and 36 are each connected with the plate 31 by a hinge portion 37 of reduced thickness. The ends of both straps 35 and 36 have a triform slot 38 that is a push fit on the respective stud 32 or 34. The bag 1 is supported on the hanger 30 by pushing the studs 32 to 34 through the holes 15 to 17 and folding the straps 35 and 36 over the top of the bag and down its rear side. The slots 38 in the straps 35 and 36 are pushed onto the studs 32 and 34 where they project through the holes 15 and 17 in the bag. The centre stud 33 projects through the centre hole 16 without any captivating strap

The plate 31 has a further opening 40 defined within a circular collar 41 projecting from both the front and rear faces of the plate. The opening 40 is located on the left-hand side of the plate and aligns with the opening 20 in the bag. The plate 31 also has a horizontally-extending hook 42 projecting from its front surface, the purpose of which will become apparent later.

At its upper end, the hanger 30 has a vertical stem 43 with a hanging hole 44 close to its upper end. Two hooked arms 45 and 46 are hinged integrally along opposite sides of the stem 42 by vertical lines 47 of reduced thickness. The rear side of the arms 45 and 46 have cooperating catches 48, which enable the arms to be clipped together when they are folded back through 90°.

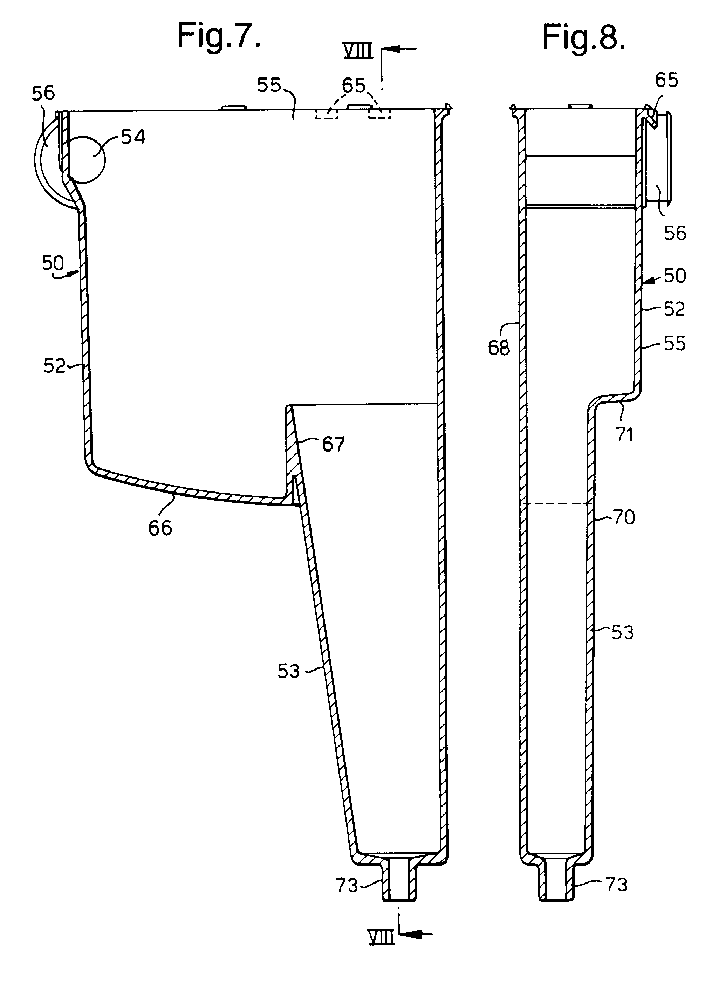

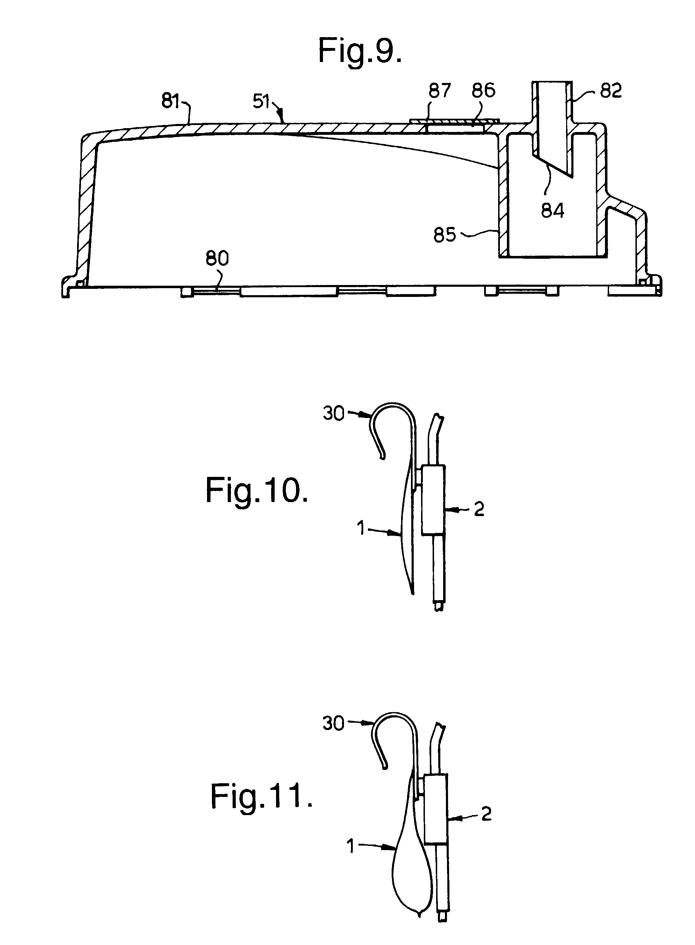

With reference now to Figures 7 to 9, the urine meter 2 is made from two components moulded from a rigid, transparent plastics material: a lower component 50 (Figures 7 and 8) in which the urine is collected, and an upper lid 51 (Figure 9). The lower component 50 is of holster shape with an upper section 52 and a narrower, lower section 53. The upper section 52 is of rectangular shape having an opening 54 at the upper left-hand corner of its rear wall 55. The opening 54 is aligned coaxially with a circular hub 56 projecting from the rear surface of the wall 55. The hub 56 has a coaxial inner and outer sleeve 57 and 58, as shown in Figure 3, the outer sleeve having an outwardly-projecting lip 59 with a tapered edge 60. The inner sleeve 57 has an internal step 61 between its outer end and its inner end. A cap 62 with an outwardly-projecting flange 63 is welded into the end of the inner sleeve 57 and traps an O-ring 64 on the step 61. The flange 63 is a snap fit within the catch 26 on the bag connector 22, with the spigot 23 extending coaxially within the inner sleeve 57 and forming a rotatable connection, which is sealed by engagement of the O-ring 64 with the outside of the spigot and the inside of the inner sleeve.

The lower component 50 has two catches 65 projecting from the upper edge of the rear wall 55, towards its right-hand side. The catches 65 are shaped to locate with the hook 42 projecting from the forward surface of the hanger 30. The meter 2 is, therefore, supported by the hanger 30, rather than by the bag 1, at its left-hand side by the pivotal coupling provided by the hub 56 and the collar 41, and at its right-hand side by engagement of the catches 65 on the hook 42.

The lower end of the upper section 52 has a floor 66, on its left-hand side, which slopes down towards the centre of the meter 2 across its width, where it is bounded by a short vertical wall 67 extending across the thickness of the meter between its front wall 68 and its rear wall 55. On the right of the wall 67, the upper section 52 opens into the lower section 53. The lower section 53 extends vertically down by a distance about equal to the height of the upper section 52. The upper end of the lower section 53 is about half the width of the upper section 52 and tapers to a smaller width at its lower end. The front wall of the lower section 53 is a continuation of the front wall 68 of the upper section 52. The rear wall 70 is stepped inwardly from the rear wall 55 of the upper section, forming a step 71 and reducing the thickness of the meter over this lower section to about half that of the upper section. The cross-section of the lower section 53 in a horizontal plane is, therefore, considerably less than that of the upper section 52 so that a unit volume of urine in the lower section produces a greater height of fluid, thereby, enabling greater resolution of volume against a vertical scale of volume graduations 72 (Figure 1). An opening 73 at the lower end of the lower section 53 receives a self-sealing sampling port 74 by which urine samples can be taken when desired.

The lid 51 has a catches 80 around its lower edge that clip onto the upper edge of the lower component 50. The roof 81 of the lid 51 has an inlet opening at its right-hand end provided by a short vertical tube 82 projecting above and below the roof. The upper end of the inlet tube 82 is connected, in use, to a urine catheter 83 or to a tube connected to a urine catheter. The lower end of the tube is bevelled at an angle of about 30° and has a rubber diaphragm or flap valve 84 attached at one side to the upper part of the bevelled surface. The flap valve 84 allows urine to enter the tube 82 from the catheter 83 but prevents reflux of urine from the meter to the catheter. The lower end of the inlet tube 82 extends within the upper part of a vertical sleeve 85, which projects down from the roof 81. The sleeve 85 acts to direct downwardly urine emerging from the inlet tube 82, so that it is directed into the lower section 53 of the meter 2. The roof 81 also has a vent opening 86 and a hydrophobic filter disc 87 covering the opening, thereby allowing gas to vent but preventing the escape of liquid.

The bag assembly may be hung for use in several different ways. The arms 45 and 46 of the hanger 30 may be folded back and locked together to form a rigid, rearwardly-extending hook, which can be hooked over any conventional bedside bag support. Alternatively, the arms 45 and 46 could be left flat and a cord or the like could be threaded through the hanging hole 44 and attached, for example, to bedding or the bed frame.

In use, urine flowing along the catheter 83 passes through the flap valve 84 and falls directly into the lower section 53 of the meter 2, where the graduations 72 enable small quantities to be measured accurately. As the meter 2 fills with urine, the level will rise above the top of the wall 67 and start to fill the upper section 53 of the meter. Because of the larger cross-section of the meter at its upper end, the larger quantities of urine can only be measured with less accuracy, but, since the larger quantities only usually need to be measured less accurately, this is not a disadvantage.

When the meter 2 is full, or when the lower part of the meter is full and an additional accurate, low volume measurement is needed, the contents of the meter are emptied into the bag 1. This is done simply by gripping the lower end of the meter 2 and swinging it up to the right in an arc about a pivot point provided by the rotatable connection of the hub 56 in the hanger 30 and the bag 1, that is, about an axis normal to the plane of the bag, so that the catches 65 on the meter are lifted off the hook 42 on the hanger. The meter 2 is, therefore, swung in a plane parallel with the plane of the bag 1 and the urine will run out of the meter, through the outlet 5 of the meter and into the bag. When the meter 2 has been emptied, it is swung back to its original position where it hangs vertically and is supported by engagement of the catches 65 on the hook 42.

This process of emptying the meter 2 into the bag 1 can be repeated as necessary. If the meter 2 is not emptied, it will overflow automatically through the openings 4 and 5 into the bag 1. As the bag 1 fills with urine, it will swell outwardly at the front and rear. The recessed lower end of the rear of the meter 2 spaces its lower end from the front of the bag 1, so that the bag can swell to a greater extent before contacting the rear of the meter, as shown in Figures 10 and 11. This enables the meter 2 to maintain a vertical position for greater volumes of urine in the bag than would be the case if the rear of the meter were flat. The coupling of the meter 2 to the bag and the location of the hanger 30 also help reduce tilting of the meter as the bag fills. This is an advantage because any movement away from the vertical will displace the surface of the urine relative to the graduations 72 and lead to errors in measurement. When the bag 1 becomes full, it is easily emptied by opening the outlet tap 14 at the bottom of the bag and allowing its contents to drain away.

The assembly of the present invention is compact and easy to use. It can provide a high accuracy even when the bag is relatively full. Because the meter is attached with the bag by a rigid, pivotal coupling, there is no risk of damage to the bag material as a result of repeated emptying of the meter.

## Claims

1. A urine bag assembly including a flexible bag (1) and a rigid meter (2) with a urine inlet (82), the meter (2) being attached with the bag (1) and having an outlet (5) connected to an inlet (4) of the bag, characterised in that the meter (2) is pivotally coupled with the bag (1) so that it can be tipped about an axis extending normal to the plane of the bag to empty its contents into the bag.

2. A bag assembly according to Claim 1, characterised in that the outlet (5) of the meter (2) connects with the inlet (4) of the bag (1) through the pivotal coupling (23, 56, 64).

3. A bag assembly according to Claim 2, characterised in that the bag (1) has a spigot (23) projecting from its inlet (4), that the meter (2) has a hub (56) surrounding the spigot, and that there is a seal (64) between the spigot (23) and the hub (56) permitting rotation of the hub relative to the spigot.

4. A bag assembly according to any one of the preceding claims, characterised in that the bag (1) includes a hanger (30).

5. A bag assembly according to Claim 4, characterised in that the hanger (30) has a plate (31) that extends on one side of the bag (1) and a member (35, 36) folded over the other side of the bag, and that the plate (31) and member (35, 36) have cooperating formations (32, 34, 38) that engage with one another through a hole (15, 17) in the bag.

6. A bag assembly according to Claim 4 or 5, characterised in that the hanger (30) has an opening (40, 41) therethrough, and that the meter (2) has a catch (59) that engages the opening and permits rotation of the meter relative to the hanger (30).

7. A bag assembly according to any one of the preceding claims, characterised in that the meter (2) has a catch (65) on its rear surface that engages with a hook (42) on the forward surface of the bag (1), and that engagement of the catch and hook limits pivoting of the meter (2) on the bag (1) in one direction.

8. A bag assembly according to any one of the preceding claims, characterised in that the rear wall (52) of the meter (2) is recessed at its lower end (70) to accommodate filling of the bag (1).

9. A bag assembly according to any one of the preceding claims, characterised in that the lower end (53) of the meter (2) has a smaller cross section than its upper end (52).

10. A bag assembly according to any one of the preceding claims, characterised in that the lower end of the bag (1) has a flexible outlet (3), and that the lower end of the bag has a recess (27) to one side of the outlet (3) so that the outlet can be bent up and retained in the recess.
